# EUROPEAN PATENT SPECIFICATION

(11) **EP 3 870 307 B1**
(45) Date of publication and mention of the grant of the patent: **12.10.2022**
(21) Application number: 19789699.6
(22) Date of filing: 23.10.2019
(51) Int. Cl.: A61Q 17/04, A61K 8/06, A61K 8/27, A61K 8/29, A61K 8/85

(54) **TOPICAL COMPOSITION COMPRISING AN INORGANIC UV-FILTER**
TOPISCHE ZUSAMMENSETZUNG MIT EINEM ANORGANISCHEN UV-FILTER
COMPOSITION TOPIQUE COMPRENANT UN FILTRE UV INORGANIQUE

(30) Priority: 25.10.2018 EP 18202512
(43) Date of publication of application: 01.09.2021
(73) Proprietor: DSM IP Assets B.V., 6411 TE Heerlen (NL)
(72) Inventor: GROOTJEN, Antonia Urmanova, 4303 Kaiseraugst (CH); JANSSEN, Anne, 4303 Kaiseraugst (CH); LAVILLA AGUILAR, Cristina, 4303 Kaiseraugst (CH); RADOMSKY, Karina, 4303 Kaiseraugst (CH)
(74) Representative: Berg, Katja
(86) International application number: PCT/EP2019/078807
(87) International publication number: WO 2020/083965

(56) References cited:
- WO-A1-2013/041515
- US-A1- 2004 132 954

## Description

The present invention relates to isopropylparabene free topical compositions comprising a specific polyester and at least one inorganic UV-filter. Furthermore, the present invention relates to the use of the combination of such polyesters and inorganic UV-filters to reduce the stickiness of compositions comprising either the polyester or the inorganic UV-filter.

Sun care products (sunscreens) have evolved considerably over the years. Today's focus is towards eliminating as much of UVA (320-400 nm) and / or UVB (280-320 nm) light as possible. Furthermore, there is an increasing demand for sun care products comprising inorganic and/ or polymeric UV-filters as they exhibit an excellent safety profile. In addition, novel regulations in view of preservatives require the development of novel formulation concepts.

Inorganic UV-filters conventionally used in the preparation of sunscreens are micronized titanium dioxide and zinc oxide. Both micropigments allow the formulation of safe and highly efficient sun care products.

A broad range of polyester based UV-filters have been disclosed in WO2013041515. However, even though providing efficient and safe UV protection, these polymeric UV-filters render cosmetic formulations sticky, which is highly undesirable by the end consumer.

It was therefore the object of the present invention to remedy the disadvantages of the prior art and to develop efficient sunscreens which exhibit a pleasant skin feel, show a reduced stickiness and a reduced transfer of the sunscreen to surfaces while respecting the latest safety regulations.

WO2013/041515 discloses polyester based UV-filters, which can be obtained from 2-(4-methoxybenzylidene)malonate dimethyl ester and a C36 dimer diol as well as to topical compositions comprising such polyester based UV-filters.

Surprisingly, it has been found that the combined use of an inorganic UV-filter and a polyester built up from C36 dimer fatty units and 2-(4-methoxybenzylidene)malonate units e.g. obtainable by polycondensation of dimethyl 2-(4-methoxybenzylidene)malonate and C36 fatty diol significantly reduced the transfer of the sun screen product onto glass surfaces such as touch screens or spectacle glasses. Furthermore, such products exhibit a significantly reduced stickiness.

In a first embodiment, the present invention relates to isopropylparabene free, topical compositions comprising an inorganic UV-filter, characterized in that the composition further comprises a polyester comprising
(i) between 20 to 50 wt.-%, based on the total weight of the polyester, of 2-(4-C₁₋₈alkoxybenzylidene)malonate units and
(ii) between 50 to 80 wt.-%, based on the total weight of the polyester, of C36 dimer fatty units, preferably C36 fatty diol units, and
with the proviso that the polyester has a hydroxyl value selected in the range of 0 to 120 mg KOH/g, a number average molecular weight (Mn) selected in the range of 1000 to 8000 g/mol and a viscosity of less than 250 Pa·s (at 25°C, measured according to ISO 3219) It is preferred, in all embodiments of the present invention, that the polyesters according to the present invention furthermore contain less than 10 wt.-%, more preferably less than 7.5 wt.-%, most preferably less than 5 wt.-% such as less than 2.5 wt.-%or even less than 1 wt.-% of polyester species with a number average molecular weight (Mn) lower than 1000 g/mol, based on the total weight of the polyester.

The term 'isopropylparabene free', as used herein refers to topical compositions which do not contain isopropylparabene.

The term 'topical' as used herein is understood here to mean external application to keratinous substances, which are in particular the skin, scalp, eyelashes, eyebrows, nails, mucous membranes and hair, preferably the skin.

In all embodiments of the present invention, preferably between 20 to 40 wt.-%, more preferably between 25 to 35 wt.-%, based on the total weight of the polyester, of 2-(4-C₁₋₈alkoxybenzylidene)malonate units are present in the polyester.

In all embodiments of the present invention, preferably between 60 to 75 wt.-%, most preferably between 65 to 75 wt.-%, based on the total weight of the polyester, of C36 dimer fatty units, preferably C36 fatty diol units are present in the polyester.

The polyesters according to the present invention are obtainable by known methods to a person skilled in the art, e.g. by polycondensation of a C₁₋₈dialkyl 2-(4-C₁₋₈alkoxybenzylidene)malonate and C36 fatty diol, optionally in the presence of small amounts of further diacids or diols such as preferably linear C₁₋₆ alkandioic acids, linear C₁₋₆ alkendioic acids and/ or linear C₁₋₆ alkanediols, such as ethyleneglycol, propyleneglycol, 1,3-propandiol, butyleneglycol, 1,6-hexandiol, malonic acid, succinic acid, fumaric acid and/ or adipic acid.

Preferably, in all embodiments according to the present invention, the polyesters according to the present invention consist essentially of 2-(4-C₁₋₈alkoxybenzylidene)malonate and C36 fatty diol units, i.e. the amounts of (i) and (ii) sum up to 100 wt.-%.

The term 'consisting essentially of' as used according to the present invention means that the total amount of the ingredients ideally adds up to 100 wt.-%. It is however not excluded that small amounts of impurities or additives may be present, with the proviso that the total amount of such impurities or additives is preferably less than 3 wt.-%, more preferably less than 2 wt.-%, most preferably less than 1 wt.-% and which are e.g. introduced via the respective raw materials.

Particularly suitable inorganic UV-filters in all embodiments of the present invention are metal powders, metal oxides or metal hydroxides which absorb light in the UVA and/ or UVB range.

The most frequently used inorganic UV-filters in sunscreens are titanium (di)oxide and zinc oxide (ZnO). These oxides are prepared in nanometric particles, which show the least reflection of visible radiation, thus producing less effect of white coloration when applied to the skin while exhibiting excellent photoprotection.

The inorganic UV-filters according to the present invention may optionally be surface treated to, for example, make the particles more hydrophobic or more dispersible in a vehicle.

Particularly preferred inorganic UV-filters according to the present invention are selected from the group consisting of titanium dioxides, zinc oxides and iron oxides, most preferably from titanium dioxide and zinc oxide, as well as mixtures thereof.

In one particular advantageous embodiment the inorganic UV-filter has a particle size which is principally useful for incorporation into sunscreen compositions.

Preferably, in all embodiments of the present invention, the titanium dioxide exhibits a primary particle size (determined by scanning electron microscope (SEM) spectroscopy) in the range of about 2 nm to 100 nm, preferably in the range of about 5 to 50 nm and a secondary particle size in the range of about 0.05 to 50 µm, preferably in the range of about 0.1 to1 µm.

The crystalline form of the titanium dioxide UV-filter may be of any crystal or amorphous type. For example, titanium dioxide may be any type of amorphous, rutil, anastase, brookite or a mixture thereof.

In a preferred embodiment, the titanium dioxide UV-filter used according to the present invention is coated (i.e. surface treated) with an organic coating such as e.g. selected from polyols, methicone, or alkyl silane. Such coatings are well known in the art. Commercially available organic coated titanium dioxides suitable according to the invention are e.g. available as Uvinul^{®} TiO₂ by BASF or Eusolex^{®} T-Avo by Merck.

In a particular preferred embodiment, the titanium dioxide UV-filter is coated with an organic coating selected from silica, silicone oils (e.g. simethicones, methicones, dimethicones, polysilicone-15) or alkyl silanes. Commercially available organic coated titanium dioxides particularly suitable according to the invention are e.g. available as Uvinul^{®} TiO₂ (INCI: trimethoxycaprylylsilane and titanium dioxide ex BASF) or Eusolex^{®} T-Avo (INCI: Titanium dioxide, Silica ex Merck).

In another advantageous embodiment the titanium dioxide UV-filter is a non-coated titanium dioxide suitable for cosmetic applications such as pyrogenic titanium dioxide (e.g. AEROXIDE P25 ex Degussa).

In a more particular embodiment of the invention, the titanium dioxide UV-filter is a double coated titanium dioxide having an inner inorganic silica coating and an outer organic coating (referred to as double coated titanium dioxide). Such coated titanium dioxides nanoparticles can be prepared according to the state of the art or are commercially available as PARSOL^{®} TX (INCI: Titanium Dioxide, Silica, Dimethicone ex DSM Nutritional Products) or as UV-Titan X195 (coated with silica and treated with a silicone oil (i.e. methicone) ex Kemira).

The inner coating of the titanium dioxide particle with inorganic silica can be prepared according to the state of the art as e.g. described in EP-A 988 853, EP-A 1 284 277, EP0988853, and US 5562897, JP 2000319128.

The inner coating layer consists of minimum 0.5 wt-%. Preferably 0.5 to 50 wt.-% inorganic silica (based on titanium dioxide), most preferably of 1 to 20 wt.%. The outer coating can be selected from the class of organic coatings such as organic polymers e.g. silicone oils (e.g. simethicones, methicones, dimethicones, polysilicone-15), alkyl silanes, olefinic acids such as stearic acid or polyols such as glycerol or organophosphonic acids. The outer coating layer consists of minimum 0.25 wt.-% based on titanium dioxide. Preferably of 0.5 to 50 wt.-%, most preferably of 0.5 to 10 wt.-%.

Other usual organic coatings can additionally be present in order to yield multiple coated (such as e.g. triple coated) titanium dioxide. The other coatings can be applied before, after or together with the second outer coating. Other additional coatings which can be used comprise organic coatings such as stearic acid, silicones (silane derivatives such as triethoxycaprylylsilane or siloxane derivatives such as methicone, dimethicone, simethicone).

In all embodiments of the present invention the titanium dioxide UV-filter is most preferably a double coated titanium dioxide (having an inner inorganic silica coating) wherein the outer coating consists of simethicone, methicone, dimethicone (also known as polydimethylsiloxane), polysilicone-15, stearic acid, glycerol and mixtures thereof, in particular of methicone, dimethicone, stearic acid or mixtures thereof. Most preferably, the outer coating consists of methicone or dimethicone, in particular of dimethicone is. Most preferred according to the invention the titanium dioxide UV-filter is UV-Titan X195 by Kemira and/or PARSOL^{®} TX by DSM Nutritional products which are titanium dioxide grades coated with silica (inner coating) and treated with a silicone oil such as in particular methicone (UV-Titan X195) or dimethicone (PARSOL^{®} TX) as outer coating. Most in particular PARSOL^{®} TX by DSM Nutritional products is used as titanium dioxide UV-filter in the compositions according to the invention.

Preferably, in all embodiments of the present invention, the zinc exhibits a primary particle size as determined by SEM spectroscopy in the range of 5 to 500 nm, preferably in the range of 10 to 450 nm, most preferably in the range of 20 to 350 nm, most preferably in the range of 30 to 300 nm.

In a preferred embodiment, the mean particle size (D50) of the zinc oxide UV-filter as determined by SEM spectroscopy is selected in the range of 10 to 300nm, preferably in the range of 25 to 250 nm, most preferably in the range of 50 to 200nm.

In a preferred embodiment, the zinc oxide UV-filter used according to the present invention is used uncoated or coated (i.e. surface treated) with an organic coating such as in particular with dimethicone, simethicone, lauric acid, jojoba esters, steaoryl glutamic acid, dimethoxydiphenylsilanetriethoxycaprylyl silane cross polymer, octyltriethoxy silane, stearic acid and/ or triethoxycaprylyl silane, preferably the organic coating is triethoxycaprylylsilane or stearic acid.

The amount of the coating is preferably selected in the range of 0.1 to 25 wt.-%, preferably in the range of 0.25 to 10 wt.-%, most preferably in the range of 0.5 to 7.5 wt.-% or even more preferably in the range of 1 to 5 wt.-%, based on the weight of the uncoated zinc oxide

Examples of suitable untreated (uncoated) and hydrophobically modified (coated) zinc oxide include but are not limited to the following Z-Cote^{®} (uncoated microfine zinc oxide) (BASF), Z-Cote^{®} HP- 1 (surface treated with Triethoxycaprylylsilane) (BASF), Sachtotec^{®} LA 10 (surface treated with lauric acid) (Sachtleben), Sachtotec^{®} (uncoated microfine zinc oxide) (Sachtleben), Spectraveil^{®} FIN, IPM, MOTG, OP, TG, TGOP (uncoated, 60% dispersion in a range of cosmetic vehicle) (ICI), Z-sperse^{®} TN (untreated, dispersion in C12-15 alkyl benzoate) (Collaborative), Z-sperse^{®} TN (untreated, dispersion in octydodecyl neopentanoate), SUNZnO-NAS (Zinc Oxide (and) Triethoxycaprylylsilane) (Sunjin).

Most preferred in all embodiments of the present invention is the use of uncoated zinc oxide or zinc oxide coated with triethoxycaprylylsilane which is e.g. commercially available as SUNZnO-NAS (Zinc Oxide (and) Triethoxycaprylylsilane) (Sunjin) or Z-Cote HP-1 (BASF).

In all embodiments of the present invention, preferably the polyesters are polyesters consisting essentially of 2-(4-C₁₋₈alkoxybenzylidene)malonate units and C36 fatty diol units obtainable by polycondensation of a C₁₋₈dialkyl 2-(4-C₁₋₈alkoxybenzylidene)malonate and C36 fatty diol.

The C₁₋₈dialkyl 2-(4-C₁₋₈alkoxybenzylidene)malonate according to the present invention are C₁₋₈dialkyl esters of 2-(4-C₁₋₈alkoxybenzylidene)malonic acid of formula (I) 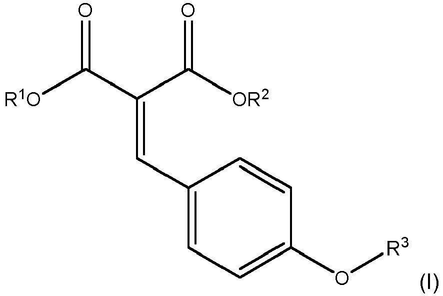 wherein R¹, R² and R³ are independently of each other selected from the group of linear or branched C₁-C₈ alkyl groups such as methyl, ethyl, propyl, isopropyl, butyl, isobutyl, tert.-butyl and ethylhexyl.

Preferably in all embodiments of the present invention R¹ = R² (i.e. R¹ and R² constitute the same C₁-C₈ alkyl group), which are even more preferably selected from the group of linear C₁-C₄ alkyl groups, most preferably from methyl or ethyl.

Even more preferably, in all embodiments of the present invention the C₁₋₈dialkyl 2-(4-C_{1- 8}alkoxybenzylidene) malonate is selected from the group of di(m)ethyl 2-(4-m(e)thoxybenzylidene)malonate, i.e. from the group consisting of
- dimethyl 2-(4-methoxybenzylidene)malonate [CAS No. 7443-25-6)],
- diethyl 2-(4-methoxybenzylidene)malonate [CAS No. 6768-23-6],
- dimethyl 2-(4-ethoxybenzylidene)malonate [CAS No. 1267390-76-0], and
- diethyl 2-(4-ethoxybenzylidene)malonate [CAS No. 27893-46-5].

The most preferred C₁₋₈dialkyl 2-(4-C₁₋₈alkoxybenzylidene)malonate in all embodiments of the present invention is dimethyl 2-(4-methoxybenzylidene)malonate which is e.g. commercially available in bulk quantities as Hostavin PR-25 from Clariant.

The C36 fatty diol (CAS No. 147853-32-5) is commercially in available in bulk quantities e.g. as Radianol 1990 from Oleon.

In all embodiments of the present invention the polyesters have a number-average molecular weight (Mn) selected in the range of 1000 to 8000 g/ mol, preferably in the range of 1500 to 4000g/mol, more preferably in the range of 1750 to 3500g/mol, most preferably in the range of 2000 to 3000 g/mol, as determined by Gel Permeation Chromatography (GPC) as described in DIN 55672-1.

The polydispersity of the polyesters according to the present invention is advantageously selected in the range of 1 to 2.5, more preferably in the range of 1.25 to 2.25, most preferably in the range of 1.5 to 2 as determined by Gel Permeation Chromatography (GPC) as outlined above.

The polyesters according to the present invention exhibit a viscosity of less than 250 Pa s, preferably of less than 150 Pa·s, more preferably of less than 100 Pa·s, most preferably of less than 75 Pa·s. Even more preferably, the viscosity of the polyesters according to the present invention is selected in the range of 10 to 100 Pa s, more preferably in the range of 25 to 75 Pa s and most preferably in the range of 40 to 60 Pa s. The viscosities as given herein are determined at 25°C according to ISO 3219.

In all embodiments of the present invention the hydroxyl value (OHV) (measured as mg KOH/g) of the polyesters according to the present invention is selected in the range of 0 to 120 mg KOH/ g, preferably in the range of 27 to 48, more preferably in the range of 30 to 45, most preferably in the range of 35 to 40 (determined titrimetrically according to ISO 4629-2-2016).

The polyesters according to the present invention are obtainable by polycondensation of a C₁₋₈dialkyl 2-(4-C₁₋₈alkoxybenzylidene)malonate and C36 fatty diol in a mol ratio selected in the range of 0.5:1.5 to 2:1 (i.e. 0.5 mol of malonate to 1.5 mol of diol to 2 mol of malonate to 1 mol of diol), preferably in the range of 0.67:1 to 1.5:1 (i.e. 0.67 mol of malonate to 1 mol of diol to 1.5 mol malonate to 1 mol of diol), most preferably in the range of 0.90:1 to 1.1:1 (i.e. 0.90 mol of malonate to 1 mol of diol to 1.1 mol of malonate to 1 mol of diol). Further particular suitable ranges include 1:1.5 to 1.5:1; 1:1.25 to 1:1; and 1:1.2 to 1:1.1 according to standard methods in the art such as e.g. by transesterification, generating the respective alcohols R¹-OH and R²-OH as by-product. Alternatively, the polyester may e.g. be prepared by esterification of the respective benzylidenemalonic acid with C36 fatty diol.

In a preferred embodiment, the polyesters according to the present invention are prepared by transesterification of a C₁₋₈dialkyl 2-(4-C₁₋₈alkoxybenzylidene)malonate with all the definitions and preferences as given herein with C36 fatty diol, preferably in the presence of an alkyl titanate, preferably tetrabutyltitanate, as transesterification catalyst. The transesterification is advantageously carried out at temperatures ranging from 100 to 180°C. Preferably, however the transesterification is carried out at relatively low temperatures, i.e. temperatures of 140°C or less, preferably of 130°C or less as this leads to a reduced discoloration of the resulting polyester. In all embodiments of the present invention is particular advantageous to carry out the transesterification at temperatures selected in the range of 100 to 140 °C, preferably 100 to 130°C, more preferably in the range of 110 to 125 °C, most preferably in the range of about 120 °C, optionally by applying vacuum. Furthermore, the transesterification is favorably carried out in the absence of oxygen (i.e. under vacuum and/ or in the presence of argon or nitrogen) as this further reduces discoloration of the final polyester.

It is further advantageous to monitor the reaction and stop it when a hydroxyl selected in the range of 25 to 50 mg KOH/g, preferably 30 to 45 mg KOH/g, most preferably 35 to 40 mg KOH/g such as in the range of 24 to 38 mg KOH/g is reached as this correlates with the stoichiometry and molecular weight range as outlined herein.

In a further advantageous embodiment, the polyester is only discharged from the reactor after cooling to ambient temperature (i.e. a temperature selected in the range of about 20 to 25 °C).

Preferably, in all embodiments of the present invention, the titanium dioxide UV filter is used in an amount selected in the range from 0.1 to 25 wt.-%, more preferably in the range from 1 to 20 wt.-%, most preferably in the range from 2.0 to 10 wt.-%, based on the total weight of the topical composition

Preferably, in all embodiments of the present invention, the zinc oxide UV filter is used in an amount selected in the range from 0.1 to 25 wt.-%, more preferably in the range from 1 to 20 wt.-%, most preferably in the range from 5 to 15 wt.-%, based on the total weight of the topical composition

Even more preferably, in all embodiments of the present invention, the total amount of the inorganic UV-filter(s) present in the topical composition is not exceeding 50 wt.-%, preferably not exceeding 30 wt.-%, most preferably not exceeding 20 wt.-%, based on the total weight of the topical composition.

The total amount of the (at least one) inorganic UV-filter in the topical compositions according to the present invention is furthermore preferably selected in the range from 0.1 to 50 wt.-%, more preferably in the range from 1 to 30 wt.-%, most preferably in the range from 2.5 to 25 wt.-%, based on the total weight of the topical composition.

The amount of the polyester in the topical compositions according to the present invention is preferably selected in the range from 0.1 to 15 wt.-%, more preferably in the range from 0.5 to 10 wt.-%, most preferably in the range from 1 to 7 wt.-%, such as in the range of 1 to 5 wt.-%, based on the total weight of the topical composition.

In a further embodiment, the present invention relates to the use of an inorganic UV-filter for reducing the transfer of a topical composition comprising a polyester according to the present invention with all the definitions and preferences as given herein to a surface such as in particular to a glass or plastic surface such as e.g. a touch screen and optionally appreciating the effect.

In another embodiment, the invention relates to a method to reduce the transfer of a topical composition comprising a polyester or an inorganic UV-filter with all the preferences and definitions as given herein to a surface such as in particular to a glass or plastic surface such as e.g. a touch screen, said method encompassing the combined use of said polyester and said inorganic UV-filter in said topical composition and optionally appreciating the effect.

In a further embodiment, the present invention relates to a method to reduce the stickiness of a topical composition comprising a polyester or an inorganic UV-filter with all the preferences and definitions as given herein, said method encompassing the combined use of said polyester and said inorganic UV-filter addition in said topical composition and optionally appreciating the effect.

Furthermore, the present invention relates to the use of an inorganic UV-filter to reduce the stickiness of a topical composition comprising a polyester as described and defined herein and optionally appreciating the effect.

As the topical compositions according to the invention are intended for topical application, they comprise a physiologically acceptable medium, that is to say a medium compatible with keratinous substances, such as the skin, mucous membranes, and keratinous fibers. In particular the physiologically acceptable medium is a cosmetically acceptable carrier.

The term 'cosmetically acceptable carrier' refers to all carriers and/or excipients and/ or diluents conventionally used in cosmetic compositions, which are well known to a person skilled in the art.

In all embodiments of the present invention, next to being isopropylparabene free, the topical compositions also advantageously do not contain isobutylparaben, phenylparaben, benzylparaben and/ or pentylparaben. Most preferably in all embodiments of the present invention, the topical compositions contain no parabenes at all, i.e. no methylparaben, ethylparaben, isopropylparabene, isobutylparaben, phenylparaben, benzylparaben and pentylparaben.

It is furthermore advantageous if the compositions according to the present invention do not contain any 3-(4-methylbenzylidene)-camphor and/ or 2-hydroxy-4-methoxy benzophenone (oxybenzone).

In another aspect, the topical compositions according to the present invention may also be free from methylisothiazolinone, chloromethylisothiazolinone, DMDM-hydantoin.

Preferred topical compositions in all embodiments of the present invention are emulsions containing an oily phase and an aqueous phase such as in particular an O/W, W/O, Si/W, W/Si, O/W/O, W/O/W multiple or a pickering emulsions.

The amount of the oily phase (i.e. the phase containing all oils and fats) present in such emulsions is preferably at least 10 wt.-%, such as in the range of 10 to 60 wt.-%, preferably in the range of 15 to 50 wt.-%, most preferably in the range of 15 to 40 wt.-%, based on the total weight of the composition.

The amount of the aqueous phase present in such emulsions is preferably at least 20 wt.-%, such as in the range from 20 to 90 wt.-%, preferably in the range from 30 to 80 wt.-%, most preferably in the range from 30 to 70 wt.-%, based on the total weight of the topical composition.

More preferably, the topical compositions according to the present invention are in the form of an oil-in-water (O/W) emulsion comprising an oily phase dispersed in an aqueous phase in the presence of an O/W- respectively Si/W-emulsifier. The preparation of such O/W emulsions is well known to a person skilled in the art and illustrated in the examples.

In an advantageous embodiment, the O/W emulsifier is a phosphate ester emulsifier. Among the preferred phosphate ester emulsifier are C₈₋₁₀ Alkyl Ethyl Phosphate, C₉₋₁₅ Alkyl Phosphate, Ceteareth-2 Phosphate, Ceteareth-5 Phosphate, Ceteth-8 Phosphate, Ceteth-10 Phosphate, Cetyl Phosphate, C6-10 Pareth-4 Phosphate, C₁₂₋₁₅ Pareth-2 Phosphate, C₁₂₋₁₅ Pareth-3 Phosphate, DEA-Ceteareth-2 Phosphate, DEA-Cetyl Phosphate, DEA-Oleth-3 Phosphate, Potassium cetyl phosphate, Deceth-4 Phosphate, Deceth-6 Phosphate and Trilaureth-4 Phosphate. A particular suitable phosphate ester emulsifier according to the invention is potassium cetyl phosphate e.g. commercially available as Amphisol^{®} K at DSM Nutritional Products Ltd Kaiseraugst.

Further advantageous O/W- or Si/W-emulsifier are selected from the list of PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate , PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, glycerylstearatcitrate, glycerylstearate (self-emulsifying), stearic acid, salts of stearic acid, polyglyceryl-3-methylglycosedistearate. Further suitable emulsifiers are sorbitan oleate, sorbitan sesquioleate, sorbitan isostearate, sorbitan trioleate, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

Further suitable are commercially available polymeric emulsifiers such as hydrophobically modified polyacrylic acid such as Acrylates/C10-30 Alkyl Acrylate Crosspolymers which are commercially available under the tradename Pemulen^{®} TR-1 and TR-2 by Noveon.

Another class of particularly suitable emulsifiers are polyglycerol esters or diesters of fatty acids also called polyglyceryl ester/ diester (i.e. a polymer in which fatty acid(s) is/ are bound by esterification with polyglycerine), such as e.g. commercially available at Evonik as Isolan GPS [INCI Name Polyglyceryl-4 Diisostearate/Polyhydroxystearate/Sebacate (i.e. diester of a mixture of isostearic, polyhydroxystearic and sebacic acids with Polyglycerin-4)] or Dehymuls PGPH available at Cognis (INCI Polyglyceryl-2 Dipolyhydroxystearate).

Also suitable are polyalkylenglycolether such as Brij 72 (Polyoxyethylen(2)stearylether) or Brij 721 (Polyoxyethylene (21) Stearyl Ether e.g. available at Croda.

The at least one O/W respectively Si/W emulsifier is preferably used in an amount of 0.5 to 10 wt.-% such as in particular in the range from0.5 to 5 wt.-% such as most in particular in the range from0.5 to 4 wt.-% based on the total weight of the composition.

Suitable W/O- or W/Si-emulsifiers are polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, polygylceryl-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

The topical compositions according to the present invention furthermore advantageously contain at least one co-surfactant such as e.g. selected from the group of mono- and diglycerides and/ or fatty alcohols. The co-surfactant is generally used in an amount selected in the range from0.1 to 10 wt.-%, such as in particular in the range from0.5 to 6 wt.-%, such as most in particular in the range from1 to 5 wt.-%, based on the total weight of the composition. Particular suitable co-surfactants are selected from the list of alkyl alcohols such as cetyl alcohol (Lorol C16, Lanette 16), cetearyl alcohol (Lanette O), stearyl alcohol (Lanette 18), behenyl alcohol (Lanette 22), glyceryl stearate, glyceryl myristate (Estol 3650), hydrogenated coco-glycerides (Lipocire Na10) as well as mixtures thereof

The topical compositions in form of O/W emulsions according to the invention can be provided, for example, in all the formulation forms for O/W emulsions, for example in the form of serum, milk or cream, and they are prepared according to the usual methods. The compositions which are subject-matters of the invention are intended for topical application and can in particular constitute a dermatological or cosmetic composition, for example intended for protecting human skin against the adverse effects of UV radiation (antiwrinkle, anti-ageing, moisturizing, anti-sun protection).

According to an advantageous embodiment of the invention the compositions constitute cosmetic composition and are intended for topical application to the skin.

Besides the inorganic UV-filters and polyesters according to the present invention, also further UV filters such as in particular UVA, UVB and/ or broadband filters may be present in the topical composition according to the present invention. These UV-filters encompass all commercially available UV-filter substances.

In a further embodiment, the present invention relates to the topical composition according to the embodiments described herein for the use as sunscreen, respectively to the use of the topical composition according to the embodiments described herein as sunscreen.

Thus, the topical compositions according to the invention are preferably light-protective preparations (sun care products), such as sun protection milks, sun protection lotions, sun protection creams, sun protection oils, sun blocks or day care creams with a SPF (sun protection factor). Of particular interest are sun protection creams, sun protection lotions, sun protection milks and sun protection preparations.

In accordance with the present invention, the compositions according to the invention may comprise further ingredients such as ingredients for skin lightening; tanning prevention; treatment of hyperpigmentation; preventing or reducing acne, wrinkles, lines, atrophy and/or inflammation; chelators and/or sequestrants; anti-cellulites and slimming (e.g. phytanic acid), firming, moisturizing and energizing, self-tanning, soothing, as well as agents to improve elasticity and skin barrier and/or further UV-filter substances and carriers and/or excipients or diluents conventionally used in topical compositions. If nothing else is stated, the excipients, additives, diluents, mentioned in the following are suitable for topical compositions according to the present invention. The necessary amounts of the cosmetic and dermatological adjuvants and additives can, based on the desired product, easily be determined by the skilled person. The additional ingredients can either be added to the oily phase, the aqueous phase or separately as deemed appropriate. The mode of addition can easily be adapted by a person skilled in the art.

The cosmetically active ingredients useful herein can in some instances provide more than one benefit or operate via more than one mode of action.

The topical cosmetic compositions of the invention can also contain usual cosmetic adjuvants and additives, such as preservatives/ antioxidants, fatty substances/ oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photoprotective effect by physically blocking out ultraviolet radiation, or any other ingredients usually formulated into cosmetic compositions.

The topical compositions according to the invention may further contain one or more emollients which soothe and soften the skin. As an example, the emollient may be dicaprylyl carbonate or C₁₂₋₁₅alkyl benzoate. Further emollients are silicone (dimethicone, cyclomethicone), vegetable oils (grape seed, sesame seed, jojoba), butters (cocoa butter, shea butter), alcohols (stearyl alcohol, cetyl alcohol), and petrolatum derivatives (petroleum jelly, mineral oil).

The cosmetic compositions according to the present invention advantageously comprise preservatives or preservative booster. Preferably, the additional preservatives respectively preservative booster is selected from the group consisting of phenoxyethanol, ethylhexylglycerin, glyceryl caprylate, caprylyl glycol, 1,2-hexanediol, propanediol, propylene glycol, hydroxyacetophenone as well as mixtures thereof. Most preferably, the topical compositions according to the present invention contains ethylhexylglycerin as preservative, optimally in admixture with phenoxyethanol. Most preferably in all embodiments of the present invention, the topical compositions comprise ethylhexylglycerin and phenoxyethanol as sole preservative.

When present, the preservative respectively preservative booster is preferably used in an amount of 0.01 to 2 wt.-%, more preferably in an amount of 0.05 to 1.5 wt.-%, most preferably in an amount of 0.1 to 1.0 wt.-%, based on the total weight of the composition. It is particularly preferred, that the cosmetic compositions according to the invention does not contain any further/ other preservatives such as e.g. parabens and/ or methylisothiazolidine.

It is furthermore beneficial if the topical compositions according to the present invention contain at least on odorant selected from the group of limonene, citral, linalool, alpha-methylionone, alpha-methylionone, geraniol, citronellol, 2-isobutyl-4-hydroxy-4-methyl tetrahydropyran, 2-tert-pentylcyclohexylacetat, 3-methyl-5-phenyl-1-pentanol, adipic ester, alpha-amylcinnamaldehyd, amylsalicylat, amylcinnamylalkohol, anisalkohol, benzoin, benzylalcohol, benzylbenzoat, benzylcinnamate, benzylsalicylate, bergamot oil, orange oil (sweet & bitter), butylphenylmethylpropional, cardamon oil, cedrol, cinnamal, cinnamylalkohol, citronellylmethylcrotonat, citrus oil, coumarin, diethylsuccinat, ethyllinalool, eugenol, isoeugenol, evernia furfuracea extract, evernia prunastri extract, farnesol, guaiac wood oil), hexylcinnamal, hexylsalicylat, hydroxycitronellal, lavender oil, lemon oil, linalylacetat, mandarin oil, menthyl PCA, methylheptenon, nutmeg oil, rosemary oil, terpineol, tonka bean oil, triethylcitrate and vanillin.

In yet another aspect, the composition may comprise sodium stearylglutamate as emulsifier and/or Silica Dimethyl Silylate and/or one or more of dibutyl adipate, dicaprylyl carbonate, C12-C15 alkylbenzoate.

In a still further aspect, the composition of the present invention may comprise one or more of alpha-lipoic acid, folic acid, phytoene, D-biotin, coenzyme Q10, alpha-glucosylrutin, carnitin, carnosin, natural and/or synthetic isoflavonoids, flavonoids, creatine, creatinine, taurine, beta-alanine, tocopheryl acetate, dihydroxyacetone; 8-hexadecene-1,16-dicarboxylic acid, glycerylglycose, (2-hydroxyethyl) urea, Vitamin E and/or derivatives thereof, hyaluronic acid and/or salts thereof, licochalcone A.

In another aspect, the composition may comprise one or more of 1,2-pentanediol, 1,2-hexanediol, 1,2-octanediol, 1,2-decanediol, 2-methyl-1,3-propanediol and/or may comprise one or more of ethanol, phenoxyethanol, ethylhexylglycerol and/or may comprise one or more of xanthan gum, crosslinked acrylate/C10-C30 alkyl acrylate polymer, vinylpyrrolidone/hexadecene copolymer and/or may comprise one or more of cetyl alcohol, stearyl alcohol, glyceryl stearate.

In another aspect, the composition may contain at least one salt of 2-phenylbenzimidazole-5-sulfonic acid.

Such cosmetic ingredients commonly used in the skin care industry, which are suitable for use in the compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the cosmetic and dermatological adjuvants and additives can - based on the desired product - easily be chosen by a skilled person in this field and will be illustrated in the examples, without being limited hereto.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

The topical compositions according to the invention in general have a pH in the range from 3 to 10, preferably a pH in the range from4 to 8 and most preferably a pH in the range from 4 to 7. The pH can easily be adjusted as desired with suitable acids such as e.g. citric acid or bases such as NaOH according to standard methods in the art.

The following examples are provided to further illustrate the compositions and effects of the present invention. These examples are illustrative only and are not intended to limit the scope of the invention in any way.

### Experimental Part

### Example 1: Preparation of the polyester

Dimethyl 2-(4-methoxybenzylidene)malonate (Hostavin PR-25, Clariant) and C36 fatty diol (Radianol 1990, Oleon) in a molar ratio of 1:1.09 were charged in a reactor (250g batch). Afterwards a catalytic amount of tetrabutyl titanate (Lehmann & Voss) was added (approx. 0.1g per 100g reaction mixture). Then the reaction mixture was heated to 120 °C. Once oligomers were formed, vacuum was applied to remove methanol (MeOH) and further built up the polymer. During this time, samples were taken, and the reaction was stopped at an OHV of 34-38 mg KOH/g. Then, the reaction mixture was cooled, and the obtained polyester was discharged obtaining a polyester having an OHV of 38.2 mg KOH/ g, a Mn of 2350 g/ mol, a polydispersity of 1.7 and a viscosity of 44.8 Pa S at 25°C (determined according to ISO 3219).

### Example 2: Application tests

The formulations (O/W emulsions) as outlined in table 1 have been prepared according to standard methods in the art.

### Transfer onto surfaces

Then the transfer resistance has been tested with the sponge test as outlined below:
- Cut a sponge cloth (Weitawip Claire from Weita AG (Art. No 279051), cellulose/ cotton mixture, 200g/m²) into pieces of 76mm x 26mm
- Tare the sponge sample
- Apply 350mg cream and distribute homogenously all over the sponge surface of 76mm x 26mm
- Weigh the sponge with the applied sample
- Tare microscope slide (glass plate 76mmx26mm)
- Put a microscope slide (glass plate) on top of the sponge and charge it with a 500g weight for 10 seconds
- Weigh the amount of cream transferred to the glass plate
- Repeat the test for each formulation 10 times to receive an average value (mean value) for each formulation

**Table 1: O/W emulsion**

| **INCI** | **Ref-1** | **Inv-1** | **Inv-2** |
|---|---|---|---|
| | wt.-% | | |
| Potassium cetyl phosphate | 1.50 | 1.50 | 1.50 |
| Cetyl Alcohol | 3.00 | 3.00 | 3.00 |
| C12-15 alkyl benzoate | 8.00 | 8.00 | 8.00 |
| Dicaprylyl Carbonate | 8.00 | 8.00 | 8.00 |
| Phenoxyethanol, Ethylhexylglycerin | 1.00 | 1.00 | 1.00 |
| Polyester of example 1 | 5.00 | 5.00 | 5.00 |
| Titanium dioxide coated with silica and dimethicone¹ | - | 5.00 | |
| Zinc oxide coated with triethoxycaprylylsilane² | - | | 5.00 |
| Xanthan gum | 0.30 | 0.30 | 0.30 |
| Disodium EDTA | 0.10 | 0.10 | 0.10 |
| Aqua | Ad 100 | Ad 100 | Ad 100 |
| Glycerin | 3.00 | 3.00 | 3.00 |
| Transfer to glass plate mean value [mg] | 28.1 | 12.7 | 12.5 |

| | | | |
|---|---|---|---|
| ¹PARSOL^{®} TX (DSM nutritional Products Ltd) ² SUNZnO-NAS (Sunjin) | | | |

As can be retrieved from table 1, an emulsion comprising the combination of an inorganic UV-filter and a polyester according to the present invention exhibited a significantly reduced amount of cream transferred to the glass surface compared to an emulsion comprising only the polyester.

### Stickiness

Afterwards the stickiness of the formulation was tested by measuring the sand adherence: as outlined below:
- The cream was applied on Schönberg plates (2mg/cm²)
- The plates were dried for 15 minutes at 40°C to form a film,
- The plate with the dried film were weighted (control plate),
- Sand was put into a petri dish,
- The plate was placed with the dried film side into the sand and a weight (500g) was put onto the plate,
- After 5 minutes the plate was taken out of the sand and weighted (sample weight)
- The amount of sand sticking to the plate was calculated (sample weight - control weight)

The results are presented below in table 2 (mean value from 6 plates).

**Table 2**

| **INCI** | **Ref-2** | **Ref-3** | **Inv-3** | **Inv-4** |
|---|---|---|---|---|
| | wt.-% | | | |
| Potassium cetyl phosphate | 1.50 | 1.50 | 1.50 | 1.50 |
| Cetyl Alcohol | 3.00 | 3.00 | 3.00 | 3.00 |
| C12-15 alkyl benzoate | 8.00 | 8.00 | 8.00 | 8.00 |
| Dicaprylyl Carbonate | 8.00 | 8.00 | 8.00 | 8.00 |
| Phenoxyethanol, Ethylhexylglycerin | 1.00 | 1.00 | 1.00 | 1.00 |
| Polyester of example 1 | - | 5.00 | - | 5.00 |
| Titanium dioxide coated with silica and dimethicone¹ | - | - | 5.00 | 5.00 |
| Xanthan gum | 0.30 | 0.30 | 0.30 | 0.30 |
| Disodium EDTA | 0.10 | 0.10 | 0.10 | 0.10 |
| Aqua | Ad 100 | Ad 100 | Ad 100 | Ad 100 |
| Glycerin | 3.00 | 3.00 | 3.00 | 3.00 |
| Sand sticking to plate [mg] | 0.52 | 0.83 | 0.58 | 0.52 |

| | | | | |
|---|---|---|---|---|
| ¹PARSOL^{®} TX (DSM nutritional Products Ltd) ² SUNZnO-NAS (Sunjin) | | | | |

As can be retrieved from the results presented in table 2, the addition of either the inorganic UV-filter or the polyester according to the present invention leads to an increase in stickiness, while the combined use resulted in a synergistically reduced stickiness (i.e. sand adherence), equivalent to the stickiness of the formulation without any UV-filter.

## Claims

1. An isopropylparabene free topical composition comprising an inorganic UV-filter **characterized in that** the composition further comprises a polyester comprising
(i) between 20 to 50 wt.-%, based on the total weight of the polyester, of 2-(4-C₁₋₈alkoxybenzylidene)malonate units and
(ii) between 50 to 80 wt.-%, based on the total weight of the polyester, C36 dimer fatty units, and
with the proviso that the polyester has a hydroxyl value selected in the range of 0 to 120 mg KOH/g, a number average molecular weight (Mn) selected in the range of 1000 to 8000 g/mol and a viscosity of less than 250 Pa s (25°C).

2. The topical composition according to claim 1, wherein the polyester is a polyester obtainable by polycondensation of a C₁₋₈dialkyl 2-(4-C₁₋₈alkoxybenzylidene)malonate and C36 fatty diol.

3. The topical composition according to claim 2, wherein the C₁₋₈dialkyl 2-(4-C₁₋₈alkoxybenzylidene)malonate is selected from the group consisting of di(m)ethyl 2-(4-(m)ethoxybenzylidene)malonate.

4. The topical composition according to any one of claims 1 to 3, wherein the polyester is obtained by transesterification of a C₁₋₈dialkyl 2-(4-C₁₋₈alkoxybenzylidene)malonate and C36 fatty diol in a mol ratio selected in the range of 1:1.5 to 1.5:1, preferably in the range of 1:1.25 to 1:1, most preferably in the range of 1:1.2 to 1:1.1.

5. The topical composition according to any one of claims 1 to 4, wherein the inorganic UV-filter is selected from the group consisting of optionally surface treated titanium dioxide and optionally surface treated zinc oxide as well as mixtures thereof.

6. The topical composition according to claim 5, wherein the titanium dioxide and the zinc oxide are surface treated.

7. The topical composition according to claim 6, wherein the titanium dioxide is double coated titanium dioxide coated with an inner silica and an outer dimethicone coating and the zinc oxide is a zinc oxide coated with triethoxycaprylylsilane.

8. The topical composition according to any one of claims 1 to 6, wherein the amount (total) of the inorganic UV-filter in the topical composition is selected in the range from 0.1 to 50 wt.-%, more preferably from 1 to 30 wt.-%, most preferably from 2.5 to 25 wt.-%, based on the total weight of the topical composition.

9. The topical composition according to any one of claims 1 to 7, wherein the amount of the polyester in the topical compositions is selected in the range from 0.1 to 15 wt.-%, preferably in the range from 0.5 to 10 wt.-%, most preferably in the range from 1 to 7 wt.-%, based on the total weight of the topical composition.

10. The topical composition according to any one of claims 1 to 9, wherein the topical composition is an emulsion containing an oily phase and an aqueous phase.

11. The topical composition according to any one of claims 1 to 10, wherein the amount of the oily phase is selected in the range from 10 to 60 wt.-%, preferably in the range from 15 to 50 wt.-%, most preferably in the range from 15 to 40 wt.-%, based on the total weight of the topical composition.

12. The topical composition according to any one of claims 1 to 11, wherein the topical composition is in the form of an oil-in-water (O/W) emulsion comprising an oily phase dispersed in an aqueous phase in the presence of an O/W emulsifier, preferably in the presence of a cetyl phosphate.

13. Use of an inorganic UV-filter to reduce the transfer of a topical composition comprising a polyester according to anyone of the preceding claims to a surface and/ or to reduce the stickiness of topical composition comprising a polyester according to anyone of the preceding claims.

14. Method to reduce the transfer of a topical composition comprising a polyester according to anyone of the preceding claims to a surface, said method encompassing the addition of an inorganic UV-filter to said topical composition.

15. Method to reduce the stickiness of a topical composition comprising a polyester or an inorganic UV-filter according to anyone of the preceding claims, said method encompassing the combined use of said polyester and said inorganic UV-filter addition in the topical composition.

## Patentansprüche

1. Isopropylparabenfreie topische Zusammensetzung, umfassend einen anorganischen UV-Filter, **dadurch gekennzeichnet, dass** die Zusammensetzung ferner einen Polyester umfasst, der
(i) zwischen 20 bis 50 Gew.-%, bezogen auf das Gesamtgewicht des Polyesters, 2-(4-C₁₋₈-Alkoxybenzyliden)malonat-Einheiten und
(ii) zwischen 50 bis 80 Gew.-%, bezogen auf das Gesamtgewicht des Polyesters, C36-Dimerfett-säuren
umfasst, und
mit der Maßgabe, dass der Polyester eine Hydroxylzahl im Bereich von 0 bis 120 mg KOH/g, zahlenmittleres Molekulargewicht (Mn) im Bereich von 1000 bis 8000 g/mol und eine Viskosität von weniger als 250 Pa·s (25 °C) aufweist.

2. Topische Zusammensetzung nach Anspruch 1, wobei es sich bei dem Polyester um einen durch Polykondensation eines C₁₋₈-Dialkyl-2-(4-C₁₋₈-alkoxybenzyliden)-malonats und eines C36-Fettdiols erhältlichen Polyester handelt.

3. Topische Zusammensetzung nach Anspruch 2, wobei das C₁₋₈-Dialkyl-2-(4-C₁₋₈-alkoxybenzyliden) malonat aus der Gruppe bestehend aus Di(m)ethyl-2-(4-(m)ethoxybenzyliden)malonat ausgewählt ist.

4. Topische Zusammensetzung nach einem der Ansprüche 1 bis 3, wobei der Polyester durch Umesterung eines C₁₋₈-Dialkyl-2-(4-C₁₋₈-alkoxybenzyliden)malonats und C36-Fettdiols in einem Molverhältnis im Bereich von 1:1,5 bis 1,5:1, vorzugsweise im Bereich von 1:1,25 bis 1:1, ganz besonders bevorzugt im Bereich von 1:1,2 bis 1,1, erhalten wird.

5. Topische Zusammensetzung nach einem der Ansprüche 1 bis 4, wobei der anorganische UV-Filter aus der Gruppe bestehend aus gegebenenfalls oberflächenbehandeltem Titandioxid und gegebenenfalls oberflächenbehandeltem Zinkoxid sowie Mischungen davon ausgewählt ist.

6. Topische Zusammensetzung nach Anspruch 5, wobei das Titandioxid und das Zinkoxid oberflächenbehandelt sind.

7. Topische Zusammensetzung nach Anspruch 6, wobei es sich bei dem Titandioxid um doppelt beschichtetes Titandioxid handelt, das mit einem inneren Siliciumdioxid-Überzug und einem äußeren Dimethicon-Überzug beschichtet ist, und es sich bei dem Zinkoxid um ein mit Triethoxycaprylylsilan beschichtetes Zinkoxid handelt.

8. Topische Zusammensetzung nach einem der Ansprüche 1 bis 6, wobei die Menge (insgesamt) des anorganischen UV-Filters in der topischen Zusammensetzung im Bereich von 0,1 bis 50 Gew.-%, weiter bevorzugt von 1 bis 30 Gew.-%, ganz besonders bevorzugt von 2,5 bis 25 Gew.-%, bezogen auf das Gesamtgewicht der topischen Zusammensetzung, gewählt ist.

9. Topische Zusammensetzung nach einem der Ansprüche 1 bis 7, wobei die Menge des Polyesters in den topischen Zusammensetzungen im Bereich von 0,1 bis 15 Gew.-%, vorzugsweise im Bereich von 0,5 bis 10 Gew.-%, ganz besonders bevorzugt im Bereich von 1 bis 7 Gew.-%, bezogen auf das Gesamtgewicht der topischen Zusammensetzung, gewählt ist.

10. Topische Zusammensetzung nach einem der Ansprüche 1 bis 9, wobei es sich bei der topischen Zusammensetzung um eine Emulsion handelt, die eine ölige Phase und eine wässrige Phase enthält.

11. Topische Zusammensetzung nach einem der Ansprüche 1 bis 10, wobei die Menge der öligen Phase im Bereich von 10 bis 60 Gew.-%, vorzugsweise im Bereich von 15 bis 50 Gew.-%, ganz besonders bevorzugt im Bereich von 15 bis 40 Gew.-%, bezogen auf das Gesamtgewicht der topischen Zusammensetzung, gewählt ist.

12. Topische Zusammensetzung nach einem der Ansprüche 1 bis 11, wobei die topische Zusammensetzung in Form einer Öl-in-Wasser(O/W)-Emulsion vorliegt, die eine in Gegenwart eines O/W-Emulgators, vorzugsweise in Gegenwart eines Cetylphosphats, in einer wässrigen Phase dispergierte ölige Phase umfasst.

13. Verwendung eines anorganischen UV-Filters zur Verringerung des Transfers einer einen Polyester umfassenden topischen Zusammensetzung nach einem der vorhergehenden Ansprüche auf eine Oberfläche und/oder zur Verringerung der Klebrigkeit einer einen Polyester umfassenden topischen Zusammensetzung nach einem der vorhergehenden Ansprüche.

14. Verfahren zur Verringerung des Transfers einer einen Polyester umfassenden topischen Zusammensetzung nach einem der vorhergehenden Ansprüche auf eine Oberfläche, umfassend die Zugabe eines anorganischen UV-Filters zu der topischen Zusammensetzung.

15. Verfahren zur Verringerung der Klebrigkeit einer einen Polyester oder einen anorganischen UV-Filter umfassenden topischen Zusammensetzung nach einem der vorhergehenden Ansprüche, umfassend die kombinierte Verwendung der Zugabe des Polyesters und des anorganischen UV-Filters in der topischen Zusammensetzung.

## Revendications

1. Composition topique exempte d'isopropylparabène comprenant un filtre UV inorganique **caractérisée en ce que** la composition comprend en outre un polyester comprenant
(i) entre 20 et 50 % en poids, sur la base du poids total du polyester, de motifs de type 2-(4-C₁₋₈-alcoxybenzylidène)malonate et
(ii) entre 50 et 80 % en poids, sur la base du poids total du polyester, de motifs gras dimères en C36, et à la condition que le polyester possède un indice d'hydroxyle choisi dans la plage de 0 à 120 mg KOH/g, un poids moléculaire moyen en nombre (Mn) choisi dans la plage de 1 000 à 8 000 g/mole et une viscosité de moins de 250 Pa.s (25 °C).

2. Composition topique selon la revendication 1, le polyester étant un polyester pouvant être obtenu par polycondensation d'un 2-(4-C₁₋₈-alcoxybenzylidène)malonate de C₁₋₈-dialkyle et d'un diol gras en C36.

3. Composition topique selon la revendication 2, le 2-(4-C₁₋₈-alcoxybenzylidène)malonate de C₁₋₈-dialkyle étant choisi dans le groupe constitué par un 2-(4-(m)éthoxybenzylidène)malonate de di(m)éthyle.

4. Composition topique selon l'une quelconque des revendications 1 à 3, le polyester étant obtenu par transestérification d'un 2-(4-C₁₋₈-alcoxybenzylidène)malonate de C₁₋₈-dialkyle et d'un diol gras en C36 en un rapport molaire choisi dans la plage de 1 : 1,5 à 1,5 : 1, préférablement dans la plage de 1 : 1,25 à 1 : 1, le plus préférablement dans la plage de 1 : 1,2 à 1 : 1,1.

5. Composition topique selon l'une quelconque des revendications 1 à 4, le filtre UV inorganique étant choisi dans le groupe constitué par du dioxyde de titane éventuellement traité en surface et de l'oxyde de zinc éventuellement traité en surface ainsi que des mélanges correspondants.

6. Composition topique selon la revendication 5, le dioxyde de titane et de l'oxyde de zinc étant traités en surface.

7. Composition topique selon la revendication 6, le dioxyde de titane étant un dioxyde de titane revêtu doublement revêtu d'un revêtement interne de silice et d'un revêtement externe de diméthicone et l'oxyde de zinc étant un oxyde de zinc revêtu par du triéthoxycaprylylsilane.

8. Composition topique selon l'une quelconque des revendications 1 à 6, la quantité (totale) du filtre UV inorganique dans la composition topique étant choisie dans la plage de 0,1 à 50 % en poids, plus préférablement de 1 à 30 % en poids, le plus préférablement de 2,5 à 25 % en poids, sur la base du poids total de la composition topique.

9. Composition topique selon l'une quelconque des revendications 1 à 7, la quantité du polyester dans la composition topique étant choisie dans la plage de 0,1 à 15 % en poids, préférablement dans la plage de 0,5 à 10 % en poids, le plus préférablement dans la plage de 1 à 7 % en poids, sur la base du poids total de la composition topique.

10. Composition topique selon l'une quelconque des revendications 1 à 9, la composition topique étant une émulsion contenant une phase huileuse et une phase aqueuse.

11. Composition topique selon l'une quelconque des revendications 1 à 10, la quantité de la phase huileuse étant choisie dans la plage de 10 à 60 % en poids, préférablement dans la plage de 15 à 50 % en poids, le plus préférablement dans la plage de 15 à 40 % en poids, sur la base du poids total de la composition topique.

12. Composition topique selon l'une quelconque des revendications 1 à 11, la composition topique étant sous la forme d'une émulsion huile-dans-eau (H/E) comprenant une phase huileuse dispersée dans une phase aqueuse en la présence d'un émulsifiant H/E, préférablement en la présence d'un phosphate de cétyle.

13. Utilisation d'un filtre UV inorganique pour réduire le transfert d'une composition topique comprenant un polyester selon l'une quelconque des revendications précédentes à une surface et/ou pour réduire l'adhésivité d'une composition topique comprenant un polyester selon l'une quelconque des revendications précédentes.

14. Procédé pour réduire le transfert d'une composition topique comprenant un polyester selon l'une quelconque des revendications précédentes à une surface, ledit procédé englobant l'ajout d'un filtre UV inorganique à ladite composition topique.

15. Procédé pour réduire l'adhésivité d'une composition topique comprenant un polyester ou un filtre UV inorganique selon l'une quelconque des revendications précédentes, ledit procédé englobant l'utilisation combinée dudit polyester et dudit ajout de filtre UV inorganique dans la composition topique.
